# EUROPÄISCHE PATENTSCHRIFT

(11) **EP 0 746 302 B1**
(45) Veröffentlichungstag und Bekanntmachung des Hinweises auf die Patenterteilung: **14.10.1998**
(21) Anmeldenummer: 95909757.7
(22) Anmeldetag: 21.02.1995
(51) Int. Cl.: A61K 7/16, A61K 7/22

(54) **MUND- UND ZAHNPFLEGEMITTEL**
MOUTH AND TOOTH CARE AGENTS
PRODUITS DE SOINS BUCCO-DENTAIRES

(30) Priorität: 02.03.1994 DE 4406746
(43) Veröffentlichungstag der Anmeldung: 11.12.1996
(73) Patentinhaber: Henkel Kommanditgesellschaft auf Aktien, 40191 Düsseldorf (DE)
(72) Erfinder: BREITZKE, Willi, D-40589 Düsseldorf (DE); GANTKE, Karl-Heinz, D-41065 Mönchengladbach (DE)
(86) Internationale Anmeldenummer: EP9500622
(87) Internationale Veröffentlichungsnummer: WO9523583

(56) Entgegenhaltungen:
- WO-A-92/05764
- WO-A-92/12701
- US-A- 5 296 215

## Beschreibung

### Gebiet der Erfindung

Die Erfindung betrifft neue Mund- und Zahnpflegemittel mit einem ausgewählten Gehalt an bestimmten Zuckertensiden und Alkyl- und/oder Alkenylsulfaten als Tensidkomponenten.

### Stand der Technik

Alkyl- und/oder Alkenyloligoglykoside und vorzugsweise Alkyloligoglucoside stellen nichtionische Tenside auf Basis nachwachsender Rohstoffe dar, die wegen ihrer ausgezeichneten anwendungstechnischen Eigenschaften und ihrer besonderen ökotoxikologischen Verträglichkeit zunehmens für die Herstellung oberflächenaktiver Mittel an Bedeutung gewinnen. Ähnliches gilt für eine weitere Gruppe von Zuckertensiden, die Fettsäure-N-alkylpolyhydroxyalkylamide, insbesondere die Fettsäure-N-alkylglucamide.

Ein Marktsegment, für welche die beiden Zuckertenside beson ders geeignet erscheinen, ist der Bereich der Mund- und Zahnpflegemittel. So werden beispielsweise in der Internationalen Patentanmeldung **WO 91/02046** (Henkel) pulverförmige Zubereitungen vorgeschlagen, die Kieselsäuren und Alkyloligoglucoside enthalten und sich für die Herstellung von Zahnpasten eignen. In der Internationalen Patentanmeldung **WO 91/02513** (Henkel) werden belaghemmende Zahnpasten offenbart, die ein antimikrobiell wirksames Biguanid, Aluminiumoxid-Trihydrat als Poliermittel und als Tensidkomponente beispielsweise Alkyloligoglucoside enthalten. Aus der Deutschen Patentanmeldung **DE-A1 41 01 505** (Henkel) sind weiterhin Mund- und Zahnpflegemittel bekannt, die Mischungen von Alkylethersulfaten und Alkyloligoglucosiden enthalten. Schließlich werden in der Japanischen Patentanmeldung **JP 1/068.312** (Shiseido) Zahnpasten mit guten Schaumeigenschaften vorgeschlagen, die Calciumhydrogenphosphat, Glycerin, Carboxymethylcellulose und Undecylglucosid beinhalten.

Von Nachteil ist jedoch, daß die genannten Abmischungen von Zuckertensiden mit anderen Tensiden hinsichtlich dermatologischer Verträglichkeit, Schaumentwicklung und Geschmacksqualität nicht alle Bedürfnisse des Marktes erfüllen.

Die Aufgabe der Erfindung hat somit darin bestanden, neue Mund- und Zahnpflegemittel zu Verfügung zu stellen, die das genannte komplexe Anforderungsprofil erfüllen.

### Beschreibung der Erfindung

Gegenstand der Erfindung sind Mund- und Zahnpflegemittel, enthaltend
a1) Alkyl- und/oder Alkenyloligoglykoside und/oder
a2) Fettsäure-N-alkylpolyhydroxyalkylamide und
b) Alkyl- und/oder Alkenylsulfate
im Gewichtsverhältnis a) : b) im Bereich von 70 : 30 bis 90 : 10.

Überraschenderweise wurde gefunden, daß Abmischungen von Alkyl- und/oder Alkenyloligoglykosiden und/oder Fettsäure-N-alkylpolyhydroxyalkylamiden einerseits und Alkyl- und/oder Alkenylsulfaten andererseits innerhalb begrenzter Gewichtsverhältnisse nicht nur eine besonders vorteilhafte dermatologische Verträglichkeit aufweisen, sondern zudem Synergien hinsichtlich der Schaumentwicklung und der geschmacklichen Qualität aufweisen. Als besonders vorteilhaft haben sich Abmischungen von Kokosalkyloligoglucosiden und Laurylsulfat im Mischungsverhätnis von 75 : 25 bis 80 : 20 erwiesen.

### Alkyl- und/oder Alkenyloligoglykoside

Alkyl- und Alkenyloligoglykoside stellen bekannte Stoffe dar, die nach den einschlägigen Verfahren der präparativen organischen Chemie erhalten werden können. Stellvertretend für das umfangreiche Schrifttum sei hier auf die Schriften **EP-A1-0 301 298** und **WO 90/3977** verwiesen. Im Sinne der Erfindung kommen Alkyl- und/oder Alkenyloligoglykoside der Formel **(I)** in Betracht, 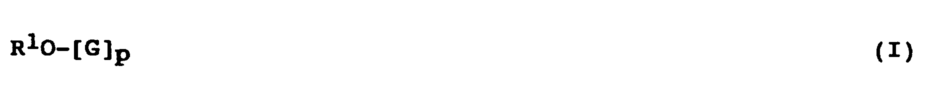 in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**.

Die Indexzahl p in der allgemeinen Formel (I) gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3).

Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Fettsäure-N-alkylpolyhydroxyalkylamide

Fettsäure-N-alkylpolyhydroxyalkylamide, die im Sinne der Erfindung als Komponente a2) eingesetzt werden können, folgen der Formel **(II)**, 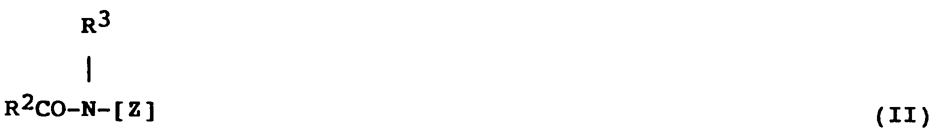 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1 985 424, US 2 016 962** und **US 2 703 798** sowie die Internationale Patentanmeldung **WO 92/06984** verwiesen. Eine Übersicht zu diesem Thema von H.Kelkenberg findet sich in **Tens. Surf.Det. 25, 8 (1988)**.

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(III)** wiedergegeben werden:

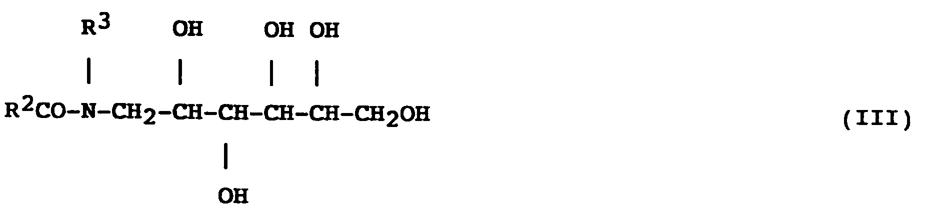

Vorzugsweise werden als Fettsäure-N-alkylpolyhydroxyalkylamide Glucamide der Formel **(III)** eingesetzt, in der R³ für Wasserstoff oder eine Amingruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(II)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

Auch die Verwendung der Fettsäure-N-alkylpolyhydroxyalkylamide ist Gegenstand einer Vielzahl von Veröffentlichungen. Aus der Europäischen Patentanmeldung **EP-A1 0 285 768** (Hüls) ist beispielsweise ihr Einsatz als Verdickungsmittel bekannt. In der Französischen Offenlegungsschrift **FR-A 1 580 491** (Henkel) werden wäßrige Detergensgemische auf Basis von Sulfaten und/oder Sulfonaten, Niotensiden und gegebenfalls Seifen beschrieben, die Fettsäure-N-alkylglucamide als Schaumregulatoren enthalten.

Gegenstand der Internationalen Patentanmeldungen **WO 92/6153; 6156; 6157; 6158; 6159** und **6160** (Procter & Gamble) sind Mischungen von Fettsäure-N-alkylglucamiden mit anionischen Tensiden, Tensiden mit Sulfat- und/oder Sulfonatstruktur, Ethercarbonsäuren, Ethersulfaten, Methylestersulfonaten und nichtionischen Tensiden. Die Verwendung dieser Stoffe in den unterschiedlichsten Wasch-, Spül- und Reinigungsmitteln wird in den Internationalen Patentanmeldungen **WO 92/6152; 6154; 6155; 6161; 6162; 6164; 6170; 6171** und **6172** (Procter & Gamble) beschrieben.

Die Alkyl- und/oder Alkenyoligoglykoside und die Fettsäure-N-alkylpolyhydroxyalkylamide, die gemeinsam die Zuckertensidkomponente a) ausmachen, können jeweils alleine oder ihrerseits in Abmischungen untereinander eingesetzt werden. Als besonders vorteilhaft haben sich dabei Mischungen im Verhältnis a1) : a2) im Bereich von 90 : 10 bis 10 : 90, vorzugswei- se 75 : 25 bis 25 : 75 und insbesondere 60 : 40 bis 40 : 60 erwiesen.

### Alkyl- und/oder Alkenylsulfate

Unter Alkyl- und/oder Alkenylsulfaten sind die Sulfatierungsprodukte primärer Alkohole zu verstehen, die der Formel **(III)** folgen, 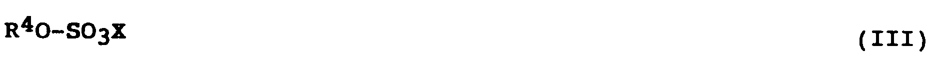 in der R⁴ für einen linearen oder verzweigten, aliphatischen Alkyl- und/oder Alkenylrest mit 6 bis 22, vorzugsweise 12 bis 18 Kohlenstoffatomen und X für ein Alkali- und/oder Erdalkalimetall, Ammonium, Alkylammonium, Alkanolammonium oder Glucammonium steht.

Typische Beispiele für Alkylsulfate, die Sinne der Erfindung Anwendung finden können, sind die Sulfatierungsprodukte von Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen, die durch Hochdruckhydrierung technischer Methylesterfraktionen oder Aldehyden aus der Roelen' schen Oxosynthese erhalten werden. Die Sulfatierungsprodukte können vorzugsweise in Form ihrer Alkalisalze, und insbesondere ihrer Natriumsalze eingesetzt werden. Besonders bevorzugt sind Alkylsulfate auf Basis von Laurylalkohol bzw. C_{12/14}-Kokosfettalkoholen in Form ihrer Natriumsalze.

Die erfindungsgemäßen Mittel enthalten die Komponenten a), d.h. die Summe aus den Einzelkomponenten a1) und a2), und b) im Gewichtsverhältnis 70 : 30 bis 90 : 10. Vorzugsweise beträgt das Verhältnis 75 : 25 bis 80 : 20.

### Tenside

Neben den genannten Tensiden, können die erfindungsgemäßen Mund- und Zahnpflegemittel in untergeordneten Mengen noch weitere, vorzugsweise anionische und/oder nichtionische Tenside enthalten.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Alkyloligoglucosidsulfate und Alkyl(ether)phosphate. Sofern die anionischen Tenside Polyglycoletherketten enthalten, können sie eine konventionelle, vorzugsweise jedoch eine eingeengte Homologenverteilung aufweisen.

Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen.

Die erfindungsgemäßen Mittel können die weiteren Tenside in Mengen von 1 bis 10, vorzugsweise 2 bis 5 Gew.-% - bezogen auf den Tensidanteil und berechnet als Feststoff - enthalten.

### Schleif- und Poliermittel

Als Schleif- und Poliermittel können die erfindungsgemäßen Mittel Kreide, Dicalciumphosphat, unlösliches Natriummetaphosphat, Aluminiumsilicat, Schichtsilicate, Hydrotalcite, Calciumpyrophosphat, feinteilige Kunstharze, Kieselsäuren, Aluminiumoxid, Aluminiumoxidtrihydrat, Talkum, Zeolithe, Magnesiumaluminiumsilicat (Veegum^{(R)}), Calciumsulfat, Magnesiumcarbonat und/oder Magnesiumoxid enthalten.

### Aromakomponenten

Neben den genannten Tensiden kommen als weitere Hilfs- und Zusatzstoffe kommen schließlich Aromakomponenten in Frage, beispielsweise Pfefferminzöl, Krauseminzöl, Anisöl, Sternanisöl, Kümmelöl, Eukalyptusöl, Fenchel, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte oder synthetisch erzeugte Komponenten dieser Öle, wie z.B. Menthol, Carvon, Anethol, Cineol, Eugenol, Zimtaldehyd, Cargophyllen, Geraniol, Citronellol, Linalool, Salven, Thymol, Terpinan, Terpinol, Methylchavicol und Methylsalicylat. Weitere geeignete Aromen sind z.B. Methylacetat, Vanillin, Ionone, Linalylacetat, Rhodinol und Piperiton. Als Süßungsmittel eignen sich entweder natürliche Zucker wie Sucrose, Maltose, Lactose und Fructose oder synthetische Süßstoffe wie z.B. Saccharin-Natriumsalz, Natriumcyclamat oder Aspartam.

### Weitere Inhaltsstoffe

Ferner kommen für den Einsatz insbesondere in Zahnpasten als Hilfs- und Zusatzstoffe Feuchthaltemittel wie z.B. Sorbit oder Glycerin, Konsistenzregler, desodorierende Wirkstoffe, Wirkstoffe gegen Mund- und Zahnerkrankungen, wasserlösliche Fluorverbindungen wie z.B. Natriumfluorid oder Natriummonofluorphosphat in Betracht.

Der Anteil der Hilfs- und Zusatzstoffe ist an sich unkritisch und richtet sich nach der Art des schließlich zu konfektionierenden Mittels. Üblicherweise wird der Anteil 5 bis 98 und vorzugsweise 80 bis 90 Gew.-% - bezogen auf die Mittel - betragen.

Eine typische Zahnpaste weist in der Regel folgende Zusammensetzung auf (Wasser ad 100 Gew.-%):
- 15 bis 25: Gew.-% Schleif- und Poliermittel,
- 30 bis 65: Gew.-% Feuchthaltemittel,
- 1 bis 10: Gew.-% Tenside,
- 1 bis 2: Gew.-% Aromastoffe und
- 0 bis 5: Gew.-% weitere Hilfsstoffe,
mit der Maßgabe, daß sich die Mengenangaben zu 100 Gew.-% ergänzen.

### Gewerbliche Anwendbarkeit

Die erfindungsgemäßen Mund- und Zahnpflegemittel zeichnen sich durch eine ausgezeichnete dermatologische Verträglichkeit auf. Sie besitzen ferner ein ausgeprägtes Schaumvermögen und gute geschmackliche Eigenschaften. Typische Beispiele für Mund- und Zahnpflegemittel, auf die sich die Erfindung erstreckt, sind Zahnpasten und Zahnputzgele.

Die folgenden Beispiele sollen den Gegenstand der Erfindung näher erläutern.

### Beispiele

### I. Eingesetzte Tenside

A) C_{8/16}-Alkyloligoglucosid (Plantaren^{(R)} APG 2000 UP, Henkel KGaA, Düsseldorf/FRG)
B) Laurinsäure-N-methylglucamid
C) Laurylsulfat-Natriumsalz (Texapon^{(R)} LS, Henkel KGaA, Düsseldorf/FRG)

### II. Anwendungstechnische Untersuchungen

Die Tensidgemische wurden in eine Standardzahnpasten-Formulierung eingesetzt (Wasser ad 100 Gew.-%):
- 21,0 g: Fällungskieselsäure (Sident^{(R)} 12 DS)
- 1,0 g: Verdickende Gelkieselsäure (Syloblanc^{(R)} 34)
- 1,2 g: Natriumcarboxymethylcellulose
- 0,1 g: Saccharin-Natriumsalz
- 0,1 g: Benzoesäure-Natriumsalz
- 1,0 g: Mundpflegearomaöl 1/074568 (Dragoco)
- 15,0 g: Sorbit (70 Gew.-%ig in Wasser)
- 25,0 g: Glycerin (86 Gew.-%ig in Wasser)
- 0,2 g: Natriumfluorid
- 2,0 g: Tensidmischung (Aktivsubstanz)
- 1,0 g: Aromaöl

*** Das Schaumvermögen wurde gemäß der Reibschaummethode in einem EHMEDA-Reibschaumgerät bestimmt [**Fette, Seifen, Anstrichmitt. 66, 955 (1964)**]. Hierzu wurden 20 g Zahnpaste in 180 g Wasser dispergiert und im Schaumzylinder auf 45°C erwärmt. Dort wurde durch 60 s Reibung mit einer vertikal rotierenden Perlonbürste bei 2600 UpM an einem zylindrisch geformten Metalldrahtgitter Schaum erzeugt. In Tabelle 1 ist das Schaumvolumen nach 0,5 min und 5 min nach Beendigung der Schaumerzeugung sowie das nach 5 min aus dem Schaum abgeschiedene Drainagewasser aufgeführt.
*** Die **Geschmacksbeurteilung** erfolgte nach dem Zähneputzen durch 5 unabhängige Testpersonen nach folgenden Kriterien:
   - ++: = Aroma vorherrschend, kein Beigeschmack
   - +: = Leichter Beigeschmack
   - -: = Intensiver Beigeschmack
   - NG: = Nachgeschmack

Die Ergebnisse sind in Tabelle 1 zusammengefaßt. Alle Prozentangaben verstehen sich als Gewichtsprozente.

**Tabelle 1**

| Zahnpastenbeurteilung | | | | | | | |
|---|---|---|---|---|---|---|---|
| **Bsp.** | **c[A] %** | **c[B] %** | **c[C] %** | **Reibschaum [ml]** | | | **Geschmack** |
| | | | | **0,5 min** | **5,0 min** | **DW ml** | |
| 1 | 70 | 0 | 30 | 850 | 690 | 70 | + |
| 2 | 80 | 0 | 20 | 950 | 780 | 70 | ++ |
| 3 | 0 | 80 | 20 | 800 | 620 | 70 | ++ |
| 4 | 40 | 40 | 20 | 880 | 670 | 70 | ++ |
| 5 | 90 | 0 | 10 | 810 | 620 | 80 | + |
| V1 | 100 | 0 | 0 | 700 | 550 | 75 | - (NG) |
| Legende: c[A] = Konzentration A DW = Drainagewasser | | | | | | | |

## Patentansprüche

1. Mund- und Zahnpflegemittel, enthaltend
a1) Alkyl- und/oder Alkenyloligoglykoside und/oder
a2) Fettsäure-N-alkylpolyhydroxyalkylamide und
b) Alkyl- und/oder Alkenylsulfate
im Gewichtsverhältnis a) : b) im Bereich von 70 : 30 bis 90 : 10.

2. Mittel nach Anspruch 1, **dadurch gekennzeichnet**, daß sie Alkyl- und/oder Alkenyloligoglykoside der Formel **(I)** enthalten, 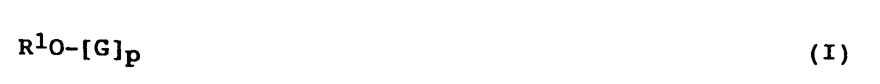 in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Mittel nach den Ansprüchen 1 und 2, **dadurch gekennzeichnet**, daß sie Fettsäure-N-alkylpolyhydroryalkylamide der Formel **(II)** enthalten, 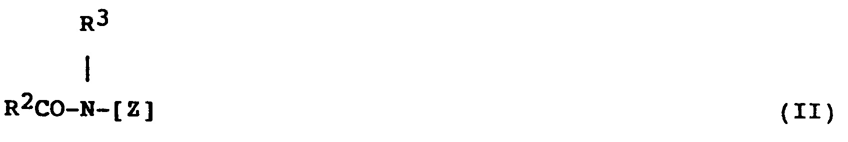 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

4. Mittel nach den Ansprüchen 1 bis 3, **dadurch gekennzeichnet**, daß sie die Komponenten a1) und a2) im Gewichtsverhältnis 90 : 10 bis 10 : 90 enthalten.

5. Mittel nach den Ansprüchen 1 bis 4, **dadurch gekennzeichnet**, daß sie Alkyl- und/oder Alkenylsulfate der Formel **(III)** enthalten, 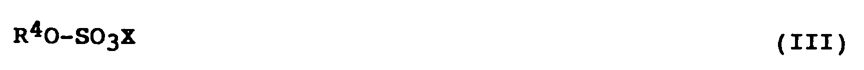 in der R⁴ für einen Alkyl- und/oder Alkenylrest mit 8 bis 22 Kohlenstoffatomen und X für ein Alkali- und/oder Erdalkalimetall, Ammonium, Alkylammonium, Alkanolammonium oder Glucammonium steht.

6. Mittel nach den Ansprüchen 1 bis 5, **dadurch gekennzeichnet**, daß es sich um Zahnpasten handelt.

7. Mittel nach den Ansprüchen 1 bis 5, **dadurch gekennzeichnet**, daß es sich um Zahnputzgele handelt.

## Claims

1. Oral hygiene and dental care preparations containing
a1) alkyl and/or alkenyl oligoglycosides and/or
a2) fatty acid N-alkyl polyhydroxyalkylamides and
b) alkyl and/or alkenylsulfates
in a ratio by weight of a) to b) of 70:30 to 90:10.

2. Preparations as claimed in claim 1, **characterized in that** they contain alkyl and/or alkenyl oligoglycosides corresponding to formula **(I):** 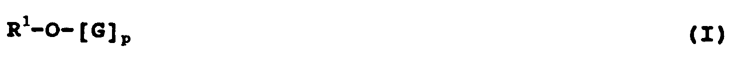 in which R¹ is an alkyl and/or alkenyl radical containing 4 to 22 carbon atoms, G is a sugar unit containing 5 or 6 carbon atoms and p is a number of 1 to 10.

3. Preparations as claimed in claims 1 and 2, **characterized in that** they contain fatty acid N-alkyl polyhydroxyalkylamides corresponding to formula **(II):** 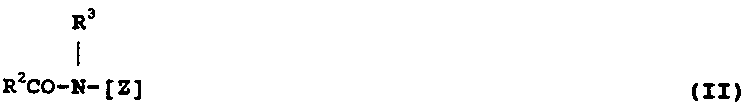 in which R²CO is an aliphatic acyl radical containing 6 to 22 carbon atoms, R³ is hydrogen, an alkyl or hydroxyalkyl radical containing 1 to 4 carbon atoms and [Z] is a linear or branched polyhydroxyalkyl radical containing 3 to 12 carbon atoms and 3 to 10 hydroxyl groups.

4. Preparations as claimed in claims 1 to 3, **characterized in that** they contain components a1) and a2) in a ratio by weight of 90:10 to 10:90.

5. Preparations as claimed in claims 1 to 4, **characterized in that** they contain alkyl and/or alkenylsulfates corresponding to formula **(III):** 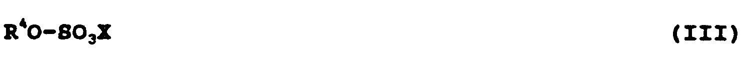 in which R⁴ is an alkyl and/or alkenyl radical containing 8 to 22 carbon atoms and X is an alkali metal and/or alkaline earth metal, ammonium, alkyl ammonium, alkanolammonium or glucammonium.

6. Preparations as claimed in claims 1 to 5, **characterized in that** they are toothpastes.

7. Preparations as claimed in claims 1 to 5, **characterized in that** they are tooth gels.

## Revendications

1. Agent de soins bucco-dentaires contenant
a1) des alkyl- et/ou alcényloligoglycosides et/ou
a2) des N-alkylpolyhydroxyalkylamides d'acides gras et
b) des sulfates d'alkyle et/ou d'alcényle
dans un rapport pondéral de a):b) compris entre 70:30 et 90:10.

2. Agents selon la revendication 1,
caractérisés en ce qu'
ils contiennent des alkyl- et/ou alcényloligoglycosides de formule (I)
R¹O - [G]ₚ (I)
dans laquelle R¹ représente un radical alkyle et/ou alcényle avec 4 à 22 atomes de carbone, G représente un radical saccharide avec 5 à 6 atomes de carbone et p représente des nombres compris entre 1 et 10.

3. Agents selon les revendications 1 et 2,
caractérisés en ce qu'
ils contiennent des N-alkylpolyhydroxyalkylamides d'acides gras de formule (II) 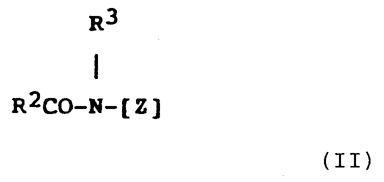 dans laquelle R²CO représente un radical acyle aliphatique avec 6 à 22 atomes de carbone, R³ représente un hydrogène, un radical alkyle ou hydroxyalkyle avec 1 à 4 atomes de carbone et [Z] représente un radical polyhydroxyalkyle linéaire ou ramifié avec 3 à 12 atomes de carbone et comportant de 3 à 10 groupes hydroxyles.

4. Agents selon les revendications 1 à 3,
caractérisés en ce qu'
ils contiennent les composants a1) et a2) dans un rapport pondéral de 90:10 à 10:90.

5. Agents selon les revendications 1 à 4,
caractérisés en ce qu'
ils contiennent des sulfates d'alkyle et/ou d'alcényle de formule (III)
R⁴O-SO₃X (III)
dans laquelle R⁴ représente un radical alkyle et/ou alcényle avec 8 à 22 atomes de carbone et X représente un métal alcalin ou alcalino-terreux, l'ammonium, un alkylammonium, un alcanolammonium ou le glucammonium.

6. Agents selon les revendications 1 à 5,
caractérisés en ce qu'
il s'agit de pâtes dentaires.

7. Agents selon les revendications 1 à 5,
caractérisés en ce qu'
il s'agit de gels de nettoyage des dents.
